Europäisches Patentamt

European Patent Office

Office européen des brevets

(19)

(11) Veröffentlichungsnummer: **0 216 991
B1**

## (12) EUROPÄISCHE PATENTSCHRIFT

(45) Veröffentlichungstag der Patentschrift:
01.03.89

(21) Anmeldenummer: 86106749.4

(22) Anmeldetag: 16.05.86

(51) Int. Cl.⁴: **C 07 C 7/08, C 07 C 11/08**

(54) Verfahren zur Trennung von paraffinischen und olefinischen C4-Kohlenwasserstoffen.

(30) Priorität: **11.09.85 DE 3532289**

(43) Veröffentlichungstag der Anmeldung:
**08.04.87 Patentblatt 87/15**

(45) Bekanntmachung des Hinweises auf die Patenterteilung:
**01.03.89 Patentblatt 89/9**

(84) Benannte Vertragsstaaten:
**AT BE DE FR GB IT NL**

(56) Entgegenhaltungen:
**DE-A- 1 916 255
DE-A- 2 359 300
DE-A- 3 318 300
DE-B- 1 908 126**

(73) Patentinhaber: **Krupp Koppers GmbH, Altendorfer Strasse 120, D-4300 Essen 1 (DE)**

(72) Erfinder: **Preusser, Gerhard, Dr. Dipl.-Chem.,
Lönsberg 24, D-4300 Essen 1 (DE)**
Erfinder: **Schulze, Martin, Krüdenscheider Weg 83,
D-5620 Velbert 15 (DE)**
Erfinder: **Emmrich, Gerd, Dipl.-Ing., Kamperfeld 21,
D-4300 Essen 1 (DE)**

## Beschreibung

Die Erfindung betrifft ein Verfahren zur Trennung von paraffinischen und olefinischen $C_4$-Kohlenwasserstoffen durch Extraktivdestillation unter Verwendung von Morpholin als selektivem Lösungsmittel, wobei die Olefine zusammen mit dem Lösungsmittel aus dem Sumpf der Extraktivdestillationskolonne abgezogen und in einer nachgeschalteten Abtriebskolonne vom Lösungsmittel abgetrennt werden.

Aus den auf der Basis von Naphtha als Einsatzprodukt arbeitenden Äthylen-Steamcrackern sowie in katalytischen Crackanlagen fallen in der chemischen Industrie große Mengen an $C_4$-Fraktionen an, die neben stärker ungesättigten $C_4$-Kohlenwasserstoffen insbesondere $C_4$-Paraffine und $C_4$-Monoolefine enthalten. Auf Grund ihrer hohen Reaktionsfähigkeit werden dabei die $C_4$-Monoolefine im zunehmenden Maße als Ausgangsstoffe für weiterführende Reaktionen eingesetzt. Aus diesem Grunde ist das Interesse der Fachwelt an einer wirtschaftlichen Auftrennung der anfallenden $C_4$-Fraktionen ständig gewachsen. Es sind deshalb bereits Verfahrensgänge entwickelt worden, bei denen zunächst aus der eingesetzten $C_4$-Fraktion das Butadien durch Gaswäsche oder Extraktivdestillation mit speziellen Lösungsmitteln abgetrennt wird, während das Isobuten durch Selektivreaktionen wie Schwefelsäureesterbildung oder die Herstellung von Methyltert.-Butylether (MTB) aus dem Einsatzprodukt entfernt wird. Die danach im Einsatzprodukt verbleibenden Komponenten Isobutan, n-Butan, Buten-1 und cis/trans-Buten 2 sind auf Grund ihrer Siedelage nur noch schwer, zum Teil aber auch gar nicht auf rein destillativem Wege zu trennen. Es besteht deshalb derzeit bei der Fachwelt ein ausgesprochenes Bedürfnis nach Verfahren, die die Trennung der genannten Verbindungen auf einfache und besonders wirtschaftliche Weise ermöglichen.

Die Verwendung von Morpholin als selektivem Lösungsmittel bei der Extraktivdestillation zur Trennung von Paraffinen und Olefinen, insbesondere auch zur Aufarbeitung von $C_4$-Fraktionen, wird bereits in der britischen Patentschrift 548 734 beschrieben. Allerdings ist bei der dort beschriebenen Arbeitsweise ein Wasserzusatz zum Morpholin in Größenordnung zwischen 5 und 35 % zwingend vorgeschrieben, weil man von der Annahme ausging, daß nur durch diesen relativ hohen Wasserzusatz die erforderliche Selektivität des Morpholins erreicht werden könne.

Auch in späteren Publikationen, wie beispielsweise der DE-OS 2 359 300, wird immer wieder von der gleichen Annahme ausgegangen und dementsprechend die Verwendung von Morpholin nur in Verbindung mit einem Wasserzusatz vorgeschlagen. Die genannte DE-OS sieht hierbei sogar vor, daß die Extraktivdestillationskolonne mit einem Wasserrückfluß betrieben werden soll.

Aus der DE-OS 1 916 255 ist ferner ein Verfahren zur Trennung von Kohlenwasserstoffgemischen, die Paraffine, Monoolefine, Diolefine sowie gegebenenfalls geringe Mengen stärker ungesättigte Kohlenwasserstoffe enthalten, durch zweistufige Extraktivdestillation bekannt, bei dem in erster Linie N-substituierte Morpholine als Lösungsmittel verwendet werden sollen. Im Hauptanspruch dieser Entgegenhaltung wird zwar auch Morpholin als Lösungsmittel aufgeführt. Der Entgegenhaltung ist aber kein Hinweis dafür zu entnehmen, daß bei Verwendung von Morpholin als Lösungsmittel die Extraktivdestillation ohne Wasserzusatz zum Lösungsmittel durchgeführt werden soll.

Die bisher übliche Arbeitsweise mit Wasserzusatz zum Morpholin führt jedoch zu schwerwiegenden wirtschaftlichen Nachteilen, die hauptsächlich auf der Tatsache beruhen, daß die hier insbesondere zur Debatte stehenden $C_4$-Kohlenwasserstoffe mit Wasser Destillationsazeotrope bilden, was in keiner dieser Veröffentlichungen erwähnt wird. Der Wassergehalt dieser Azeotrope liegt zwar nur bei 0,2-0,5 %. Die Folge davon ist jedoch, daß eine mit wasserhaltigem Morpholin durchgeführte Extraktivdestillation sowohl im Raffinat (= überwiegend gesättigte $C_4$-Kohlenwasserstoffe) als auch in Extrakt (= überwiegend olefinische $C_4$-Kohlenwasserstoffe) zu wassergesättigten Produkten führt. Weil aber die zu gewinnenden olefinischen $C_4$-Kohlenwasserstoffe praktisch ausnahmslos in katalytischen Reaktionen weiterverarbeitet werden, die mit sehr wasserempfindlichen Katalysatoren arbeiten, wie beispielsweise die Polymerisation von Buten-1 oder die Dimerisation von Buten-2, wird auf absolte Wasserfreiheit dieser Stoffe größter Wert gelegt ($H_2O$-Gehalt < 1 ppm). Im Falle der Verwendung von wasserhaltigem Morpholin muss also eine destillative oder adsorptive Trocknungsstufe der üblichen Extraktaufarbeitung nachgeschaltet werden, was sich sowohl hinsichtlich der Investitions- als auch der Energiekosten sehr nachteilig bemerkbar macht. Oftmals wird auch für den butanreichen Raffinatstrom Wasserfreiheit gefordert, weil dieses Produkt z.B. als Lösungsmittel bei wasserempfindlichen Reaktionen eingesetzt wird. Aus diesem Grunde muß dann in diesem Falle auch noch auf der Raffinatseite eine Trocknungsstufe nachgeschaltet werden. Als besonders mißlich muß hierbei die Tatsache gewertet werden, daß die zu trennenden $C_4$-Fraktionen zumeist auf Grund der vorausgegangenen Vertfahrensschritte als völlig wasserfreie Produkte vorliegen, die dann ausschliesslich wegen des Trennprozesses mit Wasser verunreinigt werden und danach in aufwenger Art und Weise wieder getrocknet werden müssen.

In der DE-PS 1 908 126 wird zwar ein Verfahren zur Trennung paraffinischer und olefinischer Kohlenwasserstoffe mittels Extraktivdestillation vorgeschlagen, bei dem wasserfreie Lösungsmittel eingesetzt werden. Hierbei handelt es sich um N-substituierte Morpholine, insbesondere um N-Formylmorpholin. Während der Einsatz derartiger Lösungsmittel bei der Trennung von nicht-aromatischen und aromatischen Kohlenwasserstoffen zu sehr guten Ergebnissen führt, ist der Einsatz dieser Lösungsmittel im vorliegenden Anwendungsfall problematisch. Dies ist in erster Linie auf die Siedelage dieser Morpholinderivate zurückzuführen, die alle Siedepunkte um 200 °C und höher aufweisen. Bei Einsatz so hochsiedender Lösungsmittel zur Trennung paraffinischer und olefinischer $C_4$-Kohlenwasserstoffe mittels Extraktivde-

stillation müssen die am Kopf der der Aufarbeitung des Extraktes dienenden Abtriebskolonne anfallenden Dämpfe durch einen Kompressor abgesaugt oder über Kälteanlagen kondensiert werden. Beide Methoden sind jedoch in der Praxis wenig beliebt und von wirtschaftlichem Nachteil, weil sie mit zusätzlichen Investitions- und Energiekosten verbunden sind und auch die Verfügbarkeit der Anlage einschränken. Die Notwendigkeit der Einschaltung solcher Hilfsmittel beruht darauf, daß sich das Lösungsmittel im Sumpf der Abtriebskolonne im Siedezustand befinden muß, um alle Kohlenwasserstoffe des Extraktes so weit als irgend möglich auszutreiben, denn der Restgehalt an Kohlenwasserstoffen in dem bei der Extraktivdestillation im Kreis geführten Lösungsmittel beeinflusst sehr wesentlich die Olefinausbeute. Bei hochsiedenden Lösungsmitteln müssen daher im Sumpf der Abtriebskolonne so hohe Temperaturen eingesetzt werden, daß das Lösungsmittel Zersetzungserscheinungen zeigt. Da das aber nicht zugelassen werden kann, wird in der Technik zumeist der Druck in der Abtriebskolonne abgesenkt und die unter Normalbedingungen· etwa um 0°C siedenden $C_4$-Kohlenwasserstoffe mittels Kompressor am Kopf der Kolonne abgesaugt.

Der Erfindung liegt deshalb die Aufgabe zugrunde, ein Verfahren zur Trennung von paraffinischen und olefinischen $C_4$-Kohlenwasserstoffen durch Extraktivdestillation zu schaffen, durch das die vorstehend geschilderten Nachteile und Schwierigkeiten der bekannten Arbeitsweisen vermieden werden. Hierbei soll das erfindungsgemässe Verfahren insbesondere auch zur Aufarbeitung von solchen $C_4$-Fraktionen eingesetzt werden können, die schwer voneinander trennbare Komponenten, wie beispielsweise das eingangs erwähnte Gemisch aus Isobutan, n-Butan, Buten-1 und cis/trans-Buten-2, enthalten.

Das der Lösung dieser Aufgabe dienende Extraktivdestillationsverfahren der eingangs genannten Art, das unter Verwendung von Morpholin als selektivem Lösungsmittel arbeitet, ist erfindungsgemäss dadurch gekennzeichnet, daß die Extraktivdestillation ohne Wasserzusatz zum Lösungsmittel durchgeführt wird und die beim Abtrieb des Lösungsmittels am Kopf der Abtriebskolonne anfallenden Dämpfe ohne Verwendung eines Kompressors und ohne Verwendung eines Kältekreislaufs durch normale Wasser- oder Luftkühlung kondensiert werden.

Das heißt, in Abkehr von der bisher von der Fachwelt vertretenen Auffassung, wird beim erfindungsgemäßen Verfahren mit wasserfreiem Morpholin als Lösungsmittel gearbeitet, und es wird auch getrennt vom Lösungsmittel kein Wasser in die Extraktivdestillationskolonne eingeleitet. Völlig unerwartet und entgegen der in der Fachliteratur bisher vertretenen Meinung hat sich nämlich gezeigt, daß das wasserfreie Morpholin für den genannten Zweck nicht nur eine ausreichende Selektivität aufweist. Es konnte außerdem vielmehr auch festgestellt werden, daß beim Einsatz von wasserfreiem Morpholin im Vergleich zur Verwendung von wasserhaltigem Morpholin eine beträchtliche Zunahme der Löslichkeit der zu trennenden $C_4$-Kohlenwasserstoffe eintritt. Dies wirkt sich zum Beispiel in der Praxis in der Weise aus, daß auch bei niedrigen Olefinkonzentrationen im Einsatzprodukt, beispielsweise 5-25% Olefin, hohe Olefinausbeuten in der Größenordnung von > 99% bei störungsfreiem Betrieb der Extraktivdestillationskolonne erreicht werden können.

Selbstverständlich ist bei Anwendung der erfindungsgemäßen Arbeitsweise eine nachträgliche Entwässerung (Trocknung) sowohl der Raffinat- als auch der Extraktsprodukte nicht erforderlich, so daß diese ohne weiteres in wasserempfindlichen Reaktionen weiter umgesetzt werden können.

Auf Grund der Siedelage von Morpholin (KP unter Normalbedingungen = 129°C) ist es beim erfindungsgemäßen Verfahren auch nicht erforderlich, die Dämpfe am Kopf der Abtriebskolonne mittels eines Kompressors abzusaugen. Es kann in dieser Kolonne vielmehr in einem Druck- und Temperaturbereich gearbeitet werden, der es gestattet, die Kopfproduktdämpfe mittels Luftkühler oder üblicher Wasserkühlung (3-3,5 bar abs. und ca. 35°C) zu kondensieren. Im Sumpf der Abtreibskolonne wird dabei eine Temperatur von 180°C nicht überschritten, und es erfolgt deshalb auch keine Zersetzung des Lösungsmittels.

Als Einsatzprodukte für das erfindungsgemäße Verfahren kommen Paraffine une Olefine enthaltende $C_4$-Fraktionen in Frage, wie sie bei verschiedenen technischen Prozessen anfallen. Typische Beispiele sind die bereits eingangs erwähnten Gemische, die nach der Entfernung des Butadiens sowie des Isobutens aus $C_4$-Fraktionen der Äthylen-Erzeugung oder der katalytischen bzw. thermischen Crackung von Kohlenwasserstoffen anfallen. Derartige Gemische enthalten, wie bereits erwähnt wurde, insbesondere Iso- und n-Butan, Buten-1 sowie cis/trans-Buten 2. Es gibt daneben aber auch technische Aufarbeitungswege, bei denen Gemische anfallen, in denen neben iso- und n-Butan nur cis/trans-Buten-2 oder nur n-Butan und cis/trans-Buten-2 enthalten. Die gemeinsame Gewinnung von Buten-1 und cis/trans--Buten-2 aus diesen Gemischen ist vor allem in den Fällen von Interesse, bei denen die drei Olefine als gleichrangige Reaktionspartner reagieren, was z.B. bei der weiteren Umsetzung zu Methyläthylketon der Fall ist. Auch die Isolierung von cis/trans-Buten-2 aus Gemischen mit n-Butan ist von besonderem Interesse, beispielsweise für die Oligomerisierung zu vorzugsweise Octen. Für alle diese Trennungen ist das erfindungsgemäße Verfahren besonders gut geeignet.

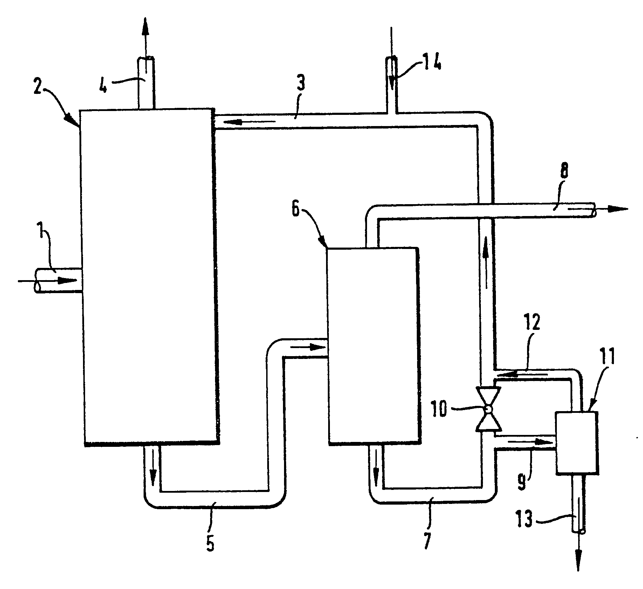

Für die Durchführung dieses Verfahrens können die für Extraktivdestillationen üblichen und allgemein gebräuchlichen Apparaturen und Anlagen eingesetzt werden. Der Verfahrensgang der erfindungsgemäßen Arbeitsweise ist dabei in dem in der Abbildung dargestellten Fließschema in vereinfachter Form wiedergegeben. Das aufzutrennende Ausgangsgemisch wird hierbei durch die Leitung 1 im flüssigen Zustand in den mittleren Teil der mit Böden oder sonstigen Einbauten versehenen Extraktivdestillationskolonne 2 eingeleitet. Durch die Leitung 3 wird das wasserfreie Morpholin am Kopf in die Extraktivdestillationskolonne 2 eingeleitet und fließt über die Einbauten dieser Kolonne herab nach unten, wobei es die Olefine aufnimmt. Die Paraffine entweichen durch Leitung 4 am Kopf der Kolonne und können in im Fließschema nicht dargestellten Kondensationseinrichtungen kondensiert werden. Das flüssige Sumpfprodukt besteht aus dem wasserfreien Morpholin und den darin gelösten Olefinen und wird durch die Leitung 5 aus der Extraktivdestillationskolonne 2 abgezogen und gelangt in die Abtriebskolonne 6, in der die Olefine destillativ vom wasserfreien Morpholin abgetrennt werden. Das wasserfreie Morpholin wird durch die Leitung 7 aus dem Kolonnensumpf entfernt und gelangt nach entsprechender Abkühlung, die im allgemeinen in einem wirtschaftlichen Wärmeverbund innerhalb der Anlage erreicht wird, über die Leitung 3 wieder in die Extraktivdestillationskolonne 2 zurück, während die Olefindämpfe über Kopf aus der Abtriebskolonne 6 entweichen und durch die Leitung 8 ihrer weiteren Verarbeitung zugeführt werden. Da sich im Laufe der Zeit im wasserfreien Morpholin Verunreinigungen anreichern können, ist im Bereich der Leitung 7 die Abzweigleitung 9 vorgesehen, durch die bei entsprechender Stellung des Ventils 10 eine Teilmenge des Morpholins zur Regeneriereinrichtung 11 gelangen kann.

Das regenerierte Morpholin wird durch die Leitung 12 wieder in den Kreislauf (Leitung 7) zurückgeführt, während die ausgeschiedenen Verunreinigungen durch die Leitung 13 aus der Regeneriereinrichtung 11 abgezogen werden. Die Leitung 14 dient schließlich der Zufuhr von frischem wasserfreien Morpholin.

Abschliessend soll die Wirkung des erfindungsgemäßen Verfahrens an Hand der nachfolgenden Verfahrensbeispiele bewiesen werden. Die Verfahrensbeispiele 1, 2 und 4 betreffen dabei die Isolierung von cis/trans-Buten-2 aus Einsatzprodukten (Ausgangsgemischen), die außer diesen Produkten n-Butan in unterschiedlichen Konzentrationen enthalten. Verfahrensbeispiel 3 betrifft die gemeinsame Gewinnung von Buten-1 und cis/trans-Buten-2 aus einem Einsatzprodukt das außer diesen Stoffen noch Iso- und n-Butan enthält.

Die wichtigsten Daten der einzelnen Verfahrensbeispiele sind in der nachfolgenden Tabelle zusammengefaßt.

## TABELLE

| Verfahrensbeispiel Nr. | 1 | 2 | 3 | 4 |
|---|---|---|---|---|
| Zsammensetzung des Ausgangsgemisches: | n-Butan 49 Gew.-%<br>Buten-2 51 Gew.-% | n-Butan 49 Gew.-%<br>Buten-2 51 Gew.-% | Isobutan 5,5 Gew.-%<br>Buten-1 45,4 Gew.-%<br>n-Buten 20,0 Gew.-%<br>Buten-2 29,1 Gew.-% | n-Butan 70 Gew.-%<br>Buten-2 30 Gew.-% |
| Selektives Lösungsmittel: | Morpholin<br>wasserfrei | Morpholin<br>wasserfrei | Morpholin<br>wasserfrei | Morpholin<br>wasserfrei |
| Menge des Ausgangsgemisches durch Leitung 1 | 10 kg/h | 10 kg/h | 10 kg/h | 10 kg/h |
| Einsatztemperatur: | 37°C | 42°C | 35°C | 35°C |
| Lösungsmittelmenge durch Leitung 3 | 120 kg/h | 135 kg/h | 150 kg/h | 130 kg/h |
| Bodenzahl in Kolonne 2 | 90 | 120 | 120 | 120 |
| Zusammensetzung der Kohlenwasserstoffe in Leitung 4 | n-Butan 99 Gew.-%<br>Buten-2 1 Gew.-% | n-Butan 99 Gew.-%<br>Buten-2 1 Gew.-% | Isobutan 20 Gew.-%<br>Buten-1 8 Gew.-%<br>n-Butan 71 Gew.-%<br>Buten-2 1 Gew.-% | n-Butan 99 Gew.-%<br>Buten-2 1 Gew.-% |
| Menge der Kohlenwasserstoffe in Leitung 4 | 4,6 kg | 4,9 kg | 2,7 kg | 6,3 kg/h |
| Bodenzahl in Kolonne 6 | 40 | 40 | 40 | 40 |
| Zusammensetzung der Kohlenwasserstoffe in Leitung 8 | n-Butan 6 Gew.-%<br>Buten-2 94 Gew.-% | n-Butan 1 Gew.-%<br>Buten-2 99 Gew.-% | Buten-1 59 Gew.-%<br>n-Butan 1 Gew.-%<br>Buten-2 40 Gew.-% | n-Butan 20 Gew.-%<br>Buten-2 80 Gew.-% |
| Menge der Kohlenwasserstoffe in Leitung 8 | 5,4 kg/h | 5,1 kg/h | 7,3 kg/h | 3,7 kg/h |
| Ausbeute: Paraffine | 93 Gew.-% | 99 Gew.-% | 97 Gew.-% | 90 Gew.-% |
| Olefine | 99 Gew.-% | 99 Gew.-% | 97 Gew.-% | 98 Gew.-% |
| Reinheit: Paraffine | 99 Gew.-% | 99 Gew.-% | 94 Gew.-% | 99 Gew.-% |
| Olefine | 94 Gew.-% | 99 Gew.-% | 99 Gew.-% | 80 Gew.-% |

## Patentanspruch

Verfahren zur Trennung von paraffinischen und olefinischen C$_4$-Kohlenwasserstoffen durch Extraktivdestillation unter Verwendung von Morpholin als selektivem Lösungsmittel, wobei die Olefine zusammen mit dem Lösungsmittel aus dem Sumpf der Extraktivdestillationskolonne abgezogen und in einer nachgeschalteten Abtriebskolonne vom Lösungsmittel abgetrennt werden, dadurch gekennzeichnet, daß die Extraktivdestillation ohne Wasserzusatz zum Lösungsmittel durchgeführt wird und die beim Abtrieb des Lösungsmittels am Kopf der Abtriebskolonne anfallenden Dämpfe ohne Verwendung eines Kompressors und ohne Verwendung eines Kältekreislaufes durch normale Wasser- oder Luftkühlung kondensiert werden.

## Claim

A process for the separation of paraffinic and olefinic C$_4$ hydrocarbons by extractive distillation using morpholine as the selective solvent, the olefins together with the solvent being taken off from the bottom of the extractive distillation column and being separated from the solvent in a downstream stripping column, characterized in that the extractive distillation is carried out without an addition of water to the solvent and the vapours arising at the top of the stripping column on stripping of the solvent are condensed by normal water cooling or air cooling without the use of a compressor and without the use of a refrigerant circulation.

## Revendication

Procédé pour séparer des hydrocarbures paraffiniques et oléfiniques en C$_4$ par distillation extractive, en utilisant de la morpholine comme solvant sélectif, les oléfines étant soutirées conjointement avec le solvant du pied de la colonne de distillation extractive et étant séparées du solvant dans une colonne de séparation montée en aval, caractérisé par le fait que la distillation extractive est effectuée sans ajouter d'eau au solvant et que les vapeurs se formant à la tête de la colonne de séparation, lors de la séparation du solvant, sont, sans utilisation d'un compresseur et sans utilisation d'un circuit de refroidissement, condensées par un refroidissement normal à l'eau ou à l'air.